# EUROPEAN PATENT SPECIFICATION

(11) **EP 3 464 620 B1**
(45) Date of publication and mention of the grant of the patent: **24.02.2021**
(21) Application number: 17728245.6
(22) Date of filing: 23.05.2017
(51) Int. Cl.: C12Q 1/6886

(54) **DETERMINATION OF GENETIC PREDISPOSITION TO AGGRESSIVE PROSTATE CANCER**
BESTIMMUNG DER GENETISCHEN PRÄDISPOSITION FÜR AGGRESSIVEN PROSTATAKREBS
DÉTERMINATION DE LA PRÉDISPOSITION GÉNÉTIQUE AU CANCER AGRESSIF DE LA PROSTATE

(30) Priority: 24.05.2016 FI 20165432
(43) Date of publication of application: 10.04.2019
(73) Proprietor: Turun yliopisto, 20014 Turun yliopisto (FI)
(72) Inventor: SIPEKY, Csilla, 20014 Turun yliopisto (FI); SCHLEUTKER, Johanna, 20014 Turun yliopisto (FI); WESTERMARCK, Jukka, 20014 Turun yliopisto (FI)
(74) Representative: Kolster Oy Ab
(86) International application number: PCT/FI2017/050383
(87) International publication number: WO 2017/203100

(56) References cited:
- WO-A2-2013/070933
- JIANYING ZHANG: "HapMap-based study of CIP2A gene polymorphisms and HCC susceptibility", ONCOLOGY LETTERS, 23 May 2012 (2012-05-23), XP055394858, GR ISSN: 1792-1074, DOI: 10.3892/ol.2012.728 cited in the application
- VAARALA MARKKU H ET AL: "CIP2A expression is increased in prostate cancer", JOURNAL OF EXPERIMENTAL & CLINICAL CANCER RESEARCH, BIOMED CENTRAL LTD, LONDON UK, vol. 29, no. 1, 21 October 2010 (2010-10-21), page 136, XP021083420, ISSN: 1756-9966, DOI: 10.1186/1756-9966-29-136 cited in the application
- AFFYMETRIX: "Data Sheet Affymetrix(R) Genome-Wide Human SNP Array 6.0", INTERNET CITATION, 2007, pages 1-4, XP002525407, Retrieved from the Internet: URL:http://www.affymetrix.com/support/tech nical/datasheets/genomewide_snp6_datasheet .pdf [retrieved on 2009-04-10]
- J. L. BEEBE-DIMMER ET AL: "The HOXB13 G84E Mutation Is Associated with an Increased Risk for Prostate Cancer and Other Malignancies", CANCER EPIDEMIOLOGY, BIOMARKERS AND PREVENTION., vol. 24, no. 9, 24 June 2015 (2015-06-24), pages 1366-1372, XP055394935, US ISSN: 1055-9965, DOI: 10.1158/1055-9965.EPI-15-0247
- PRADIP DE ET AL: "Oncogenic nexus of cancerous inhibitor of protein phosphatase 2A (CIP2A): an oncoprotein with many hands", ONCOTARGET, vol. 5, no. 13, 15 July 2014 (2014-07-15), pages 4581-4602, XP055394897, DOI: 10.18632/oncotarget.2127

### SEQUENCE LISTING

<110> Turun yliopisto
<120> DETERMINATION OF GENETIC PREDISPOSITION TO AGGRESSIVE PROSTATE CANCER
<130> 2160249PC
<160> 4
<170> PatentIn version 3.5
<210> 1
   <211> 15
   <212> DNA
   <213> Homo sapiens
<220>
   <221> misc_feature
   <222> (5)..(5)
   <223> n is c or t
<400> 1
   gcctncaaag taacc 15
<210> 2
   <211> 15
   <212> DNA
   <213> Homo sapiens
<220>
   <221> misc_feature
   <222> (11)..(11)
   <223> n is g or a
<400> 2
   ggttactttg naggc 15
<210> 3
   <211> 15
   <212> DNA
   <213> Homo sapiens
<220>
   <221> misc_feature
   <222> (5)..(5)
   <223> n is c or t
<400> 3
   gtttngagca tggaa 15
<210> 4
   <211> 15
   <212> DNA
   <213> Homo sapiens
<220>
   <221> misc_feature
   <222> (11)..(11)
   <223> n is g or a
<400> 4
   ttccatgctc naaac 15

## Description

### FIELD OF THE INVENTION

The present invention relates to prostate cancer-associated genetic markers. More specifically, the invention relates to a method of predicting, diagnosing and/or prognosing prostate cancer, especially a clinically relevant, aggressive form of prostate cancer. The invention also relates to different uses of said method, and a test kit for use in said method.

### BACKGROUND OF THE INVENTION

Prostate cancer is the second most common cancer in men worldwide. An estimated 1.1 million men were diagnosed with prostate cancer in 2012, accounting for 15% of the cancers diagnosed in men, with almost 70% of the cases (759,000) occurring in more developed regions. With an estimated 307,000 deaths in 2012, prostate cancer is the fifth leading cause of male cancer deaths (6.6% of the total men deaths) worldwide.

Early detection and treatment can significantly improve prostate cancer survival. The most frequently used test to screen for prostate cancer, namely the prostate specific antigen (PSA) blood test, is effective in detecting early stage prostate cancer. Attempts to further increase the sensitivity and accuracy of PSA screening have led to the development of modified assays detecting alternative molecular forms of the molecule, including intact (iPSA), free PSA (fPSA) and total (tPSA). Some assays further incorporate the measurement of human kalligrein 2 (hK2). Despite the improvements in the PSA screening assays, they are not able to accurately distinguish between aggressive, potentially fatal cancers from clinically insignificant cancers that would have caused no symptoms or harm in the patient. On the other hand, the PSA screening assays may also miss some harmful cancers. Universally however, PSA screening has led to over-diagnosis of prostate cancer as majority of prostate cancers progress slowly and only possess a low risk of prostate cancer-related morbidity and mortality.

Because of the lack of effective prognostic markers, most men with PSA screen-detected prostate cancer undergo, often unnecessary, radical prostatectomy or radiotherapy that have side effects such as sexual impotence, urinary incontinence and bowel problems. Thus, prognostic factors for the severity of the disease would be highly beneficial to avoid overtreatment of the less aggressive and slowly growing forms of prostate cancer, and to specifically target the more radical forms of therapy to the aggressive forms of prostate cancer.

Genetic factors have been estimated to account for up to 58% of the prostate cancer risk, and to date over 100 susceptibility loci, each being a common variant with low penetrance, have been associated with prostate cancer. In subjects of Nordic descent, the highest genetic impact on the prostate cancer risk has been attributed to a rare recurrent missense mutation, namely rs138213197/G84E, in *HOXB13,* a homeobox transcription factor gene that is important in prostate development. Accordingly, Published International Application WO 2013/070933 discloses a method of identifying subjects having said missense mutation in *HOXB13* as subjects having an increased propensity to develop prostate cancer. However, to again avoid overestimation of the risk of prostate cancer and at the same time raising unnecessary concern among the studied individuals, better markers are needed for more accurate prediction of the appearance of the prostate cancer and especially the more aggressive forms of prostate cancer. Discovery of such markers, genetic or other, would be highly beneficial to determine the risk of prostate cancer and especially the risk of aggressive prostate cancer in the overall population or for example in individuals who belong to families with a high incidence of prostate cancer.

Cancerous inhibitor of protein phosphatase 2A (CIP2A), also known as KIAA1524 and p90, is a recently identified human oncoprotein, whose expression is known to be increased at high frequency in most of the human cancer types, including prostate cancer, where it has been associated with poorly differentiated and high-risk tumours (Vaarala et al., J Exp Clin Cancer Res. 2010; 29:136). However, mechanisms inducing CIP2A expression in cancer still remain largely unknown. So far, CIP2A has been studied mainly on the expression and protein level, while only one study has concerned its potential role as a cancer susceptibility gene. In that study (Li et al., Oncol Lett. 2012; 4:358-364), however, neither of the analysed mutations (rs2278911 and rs4855656) were found to be associated with the risk of hepatocellular carcinoma (HCC). The main finding of the study was that C carriers of rs2278911 exerted synergetic effect with hepatitis B and C virus infection on risk of HCC in Han Chinese population.

In accordance with the above, there is an identified need for predictive, diagnostic and prognostic markers that can preferentially differentiate between prostate cancers with lower clinical significance, including those that are localized and/or slowly growing, of those prostate cancers that are likely to progress to an advanced or lethal disease if left untreated and that cause most of the morbidity and mortality related to the disease. Such markers would be needed for example for directing the most intensive and generally most harmful forms of therapy to those prostate cancer patients who have the highest prostate cancer related morbidity and mortality and hence the highest need of therapy.

Furthermore, as developing new medical drugs and therapies for subjects suffering from prostate cancer is also challenged by the highly variable cancer-related morbidity and mortality, markers allowing more accurate selection of subjects with the more aggressive forms of prostate cancer for therapeutic trials are crucially needed to facilitate the development of new or alternative treatment strategies especially for the aggressive forms of the disease.

### BRIEF DESCRIPTION OF THE INVENTION

In one aspect, the present invention provides a method of analyzing a subject for prostate cancer-associated markers, wherein the method comprises testing a sample obtained from said subject for *HOXB13* rs138213197 and *CIP2A* rs2278911, and wherein the presence of at least one *HOXB13* rs138213197 T allele and at least one *CIP2A* rs2278911 T allele indicates that said subject has an increased risk of having or developing prostate cancer, which in some further embodiments is aggressive prostate cancer, such as castration-resistant prostate cancer. In some still further embodiments, said increased risk of having or developing prostate cancer indicates earlier than an average onset of clinically relevant prostate cancer, and in some even further embodiments said subject has an earlier diagnosis of benign prostate hyperplasia and/or is a Caucasian male.

The present method may be used for various purposes including but not limited to those selected from the group consisting of determining a subject's risk of prostate cancer, determining a subject's susceptibility to prostate cancer, determining a subject's risk of having or developing prostate cancer, diagnosing prostate cancer in a subject, prognosing prostate cancer in a subject, stratifying a subject into a high-risk or low-risk group for prostate cancer, stratifying a subject for monitoring of onset of prostate cancer, stratifying a subject for active surveillance for the progression of prostate cancer, stratifying a subject for radiotherapy or prostatectomy, stratifying a subject for clinical studies, and population screening for prostate cancer.

In another aspect, the present invention provides use of a kit in the present method and in any embodiments thereof. Said kit comprises a first binding body for testing said sample for *HOXB13* rs138213197 and a second binding body for testing said sample for *CIP2A* rs2278911.

In some embodiments of the present use of the kit, said first binding body is for specifically determining the presence or absence of *HOXB13* rs138213197 (T) and/or said second binding body is for specifically determining the presence or absence of *CIP2A* rs2278911 (T). Alternatively or additionally, the kit may comprise further first binding bodies and/or further second binding bodies for specifically determining the presence or absence of *HOXB13* rs138213197 (C) and/or *CIP2A* rs2278911 (C), respectively.

In some further embodiments of the present kit, said first and second binding bodies are selected, independently from each other, from the group consisting of nucleic acid primers, nucleic acid probes, aptamers, antibodies or antibody fragments, peptides, receptors, and enzymes such as restriction enzymes.

Other objectives, aspects, embodiments, details and advantages of the present invention will become apparent from the following figures, detailed description, examples, and dependent claims.

### DETAILED DESCRIPTION OF THE INVENTION

An object of the present invention is to provide a method for prediction, diagnostics and/or predictive prognostics of prostate cancer, especially aggressive prostate cancer.

This object may be achieved, at least partly, by a method of determining the presence of two germline mutations, namely *HOXB13* rs138213197 (T) and *CIP2A* rs2278911 (T), which possess a surprising synergistic effect in the increased occurrence of prostate cancer, especially aggressive prostate cancer, in subjects bearing the two mutations simultaneously. The synergistic effect utilized herein manifests itself in that the predictive, diagnostic and/or prognostic potential of the markers when occurring simultaneously is better than the sum of the parts. While the *HOXB13* rs138213197 (T) has predictive, diagnostic and/or prognostic potential (although to a lesser extent) when used alone, the *CIP2A* rs2278911 (T) has no such potential when used as a sole marker. Such synergistic markers are considerably more difficult to find compared to markers with direct and predictable effect on occurrence or severity of a disease and are, accordingly, typically discovered accidentally.

As used herein, the term "prostate cancer" refers to any cancer of the prostate gland, small walnut-shaped organ that produces the seminal fluid that nourishes and transports sperm, in which cells of the prostate mutate and begin to multiply out of control. The term includes not only adenocarcinomas, which develop from the gland cells and are the most common types of prostate cancer, but also rare prostate cancers including, but not limited to, sarcomas, small cell carcinomas and other neuroendocrine tumours, and transitional cell carcinomas. The term "prostate cancer" also includes all stages, categories, subcategories, and stage groupings of prostate cancer regardless of the staging system used. As way of example, the most common staging system for prostate cancer is the TNM system. The TNM system is based on the size and/or extent (reach) of the primary tumor (accounts for the letter "T" in TNM), the amount of spread to nearby lymph nodes (accounts for the letter "N"), and the presence of metastasis (accounts for the letter "M") or secondary tumors formed by the spread of cancer cells to other parts of the body.

Prostate cancer typically grows slowly and initially remains confined to the prostate gland, where it may not cause serious clinical harm. While the slowly growing types of prostate cancer may need minimal or no treatment, some other types of prostate cancer are more aggressive and can spread to other body parts, i.e. metastasize.

As used herein, the terms "aggressive prostate cancer" and "aggressive form of prostate cancer" are interchangeable and mean prostate cancers that are related with a higher cancer-related morbidity and/or mortality compared with the slowly growing, localized forms of the cancer. Characteristically the aggressive forms of prostate cancer grow faster and/or have or will develop the ability to metastasize. Examples of aggressive prostate cancers also include, but are not limited to, castration-resistant prostate cancers, poorly differentiated prostate cancers, high grade prostate cancers, and prostate cancers having a Gleason score of 8 or greater.

Since prostate cancer growth is often driven by male sex hormones called androgens, which include testosterone, a common treatment option for prostate cancer is to lower the levels of androgens in a man's body by chemical or surgical means. This type of treatment may be called hormone therapy, androgen-deprivation therapy, or castration treatment. Eventually, many metastatic prostate cancers become resistant to treatment and resume growth despite castration treatment. Prostate cancer, which is no longer responsive to castration treatment, is herein referred to by the term "castration-resistant prostate cancer" (CRPC). As used herein, the term "screen-detected prostate cancer" refers to prostate cancer, which has been detected through increased blood level of prostate-specific antigen (PSA). Many of the screen-detected prostate cancers are clinically insignificant and would have remained undetected during life in the absence of screening. Thus, the PSA screening may lead to over-diagnosis of prostate cancer.

As used herein, the term "clinically-detected prostate cancer" refers to a prostate cancer which has been detected through clinical symptoms and hence indicating cancer-related morbidity. The terms "clinically significant prostate cancer" and "clinically relevant prostate cancer" are interchangeable and, in addition to clinically-detected prostate cancers, also comprise aggressive, high-grade, large-volume, and/or non-organ defined prostate cancers which have progressed or are likely to progress to an advanced or lethal disease if left untreated, as well as prostate cancers related to significant morbidity or mortality of the disease.

As used herein, the term "benign prostatic hyperplasia" (BPH), also known as benign prostatic hypertrophy, refers to a common noncancerous enlargement of the prostate gland in aging men. Generally, BPH is considered neither as a premalignant lesion nor a precursor of carcinoma.

As used herein, the term "subject" refers to a mammalian male, preferably a human male. The subject can belong to any race or population, but is preferably a Caucasian male. In some embodiments, the subject can be one who has not been previously diagnosed as having prostate cancer, and who may or may not exhibit one or more signs or symptoms of prostate cancer. In some other embodiments, the subject can be one who has been previously diagnosed with prostate cancer, or identified as a subject in need of treatment for prostate cancer. He may also have already undergone treatment for prostate cancer or for one or more signs or symptoms of prostate cancer. Herein, the terms "subject", "individual", and "patient" are interchangeable.

In some embodiments, said subject is suspected to have or be at risk of having or developing prostate cancer, or is to be analysed or determined for risk of prostate cancer, genetic predisposition or susceptibility to prostate cancer, the presence or absence of prostate cancer, or the progression of prostate cancer, or is to be diagnosed for prostate cancer.

As used herein, the term "carrier status" refers to the presence or absence of a specific allele of a gene in an individual's genome.

As used herein, the term "allele" refers to any one of a series of two or more alternative forms of a gene that occupy the same position or locus on a chromosome. Different alleles are said to be polymorphic.

As used herein, the term "major allele" or "prevalent allele" refers to the allele that is found in the majority of individuals within a given population, whereas the term "minor allele" or "uncommon allele" refers to the allele that is found in the minority of individuals within a given population. With reference to genes having an impact on disease susceptibility, minor alleles are typically alleles associated with a risk of or predisposition to said disease.

The term "heterozygous" refers to individuals with one major allele and one minor at a specific locus. The term "minor homozygous" refers to individuals with two minor alleles at a specific locus, whereas the term "major homozygous" refers to individuals with two major alleles at a specific locus.

Accordingly, the term "carrier" refers to a subject who carries a specific allele in his genome, either in homozygous or heterozygous form.

As used herein, the term "single nucleotide polymorphism" (SNP) refers to a polymorphism that occurs at a single nucleotide position in a nucleotide sequence for which two or more alternative alleles are present in a given population. SNPs include single nucleotide insertions, deletions, and substitutions, which may, but need not, result in detectable differences in gene expression or protein function. A substitution that changes a codon coding for one amino acid to a codon coding for a different amino acid may be referred to as a missense mutation. As is well known in the art, each SNP is identifiable by a unique SNP rs ID number designated by the National Center for Biotechnology Information (NCBI).

As used herein, the term "polymorphism" refers to variation caused by the alternative alleles among individuals in a population but which variations can also be detected by other means and at different molecular levels than by detecting the sequence variation at the gene-level. Alternative molecular levels at which polymorphisms can be evaluated or determined instead of or in addition to the analysing the corresponding gene alleles can be selected from the group consisting of, for example, the corresponding gene-expression products including mRNA and/or protein.

As used herein, the term *"HOXB13"* or *"Homeobox B13"* refers to a homeobox transcription factor gene located in a cluster of HOX genes, the cytogenetic location of which is 17q21.32 and which encodes the HOXB13 protein.

As used herein, the term *"HOXB13* rs138213197" refers to a polymorphic genomic sequence GCC T[C/T]C AAA GTA ACC (SEQ ID NO:1) at NC_000017.11:g.48728343 position (GRCh38.p2), which manifests itself at the expression level as the mRNA sequence GGT TAC TTT G[G/A]A GGC (SEQ ID NO:2). The "C allele" of the C/T SNP of rs138213197 is the major *HOXB13* allele, and it may also be referred to by the terms "non-risk allele", "non-disease-associated allele", and *"HOXB13* rs138213197 (C)". Accordingly, the "T allele" of the C/T SNP of rs138213197 is the minor *HOXB13* allele, and it may be referred to by the terms "risk allele", "disease-associated allele", *"HOXB13* rs138213197 (T)", *"HOXB13* T allele", and the like.

The change of cytosine for thymine at NC_000017.11:g.48728343 position (GRCh38.p2) in *HOXB13* results in amino acid variation denoted as "G84E", wherein position 84 encodes a glutamic acid (E) instead of a glycine (G). Accordingly, the G84E variant of *HOXB13* may be referred to by the terms "risk variant" and "disease-associated variant". As used herein, the terms *"HOXB13* G84E", *"HOXB13* rs138213197 (T)", and *"HOXB13* T allele" are interchangeable.

As used herein, the term *"CIP2A"* refers to a gene that encodes the human oncoprotein CIP2A, also known as KIAA1524 or p90, the cytogenetic location of which is 3q13.13.

As used herein, the term *"CIP2A* rs2278911" refers to a polymorphic genomic sequence GTT T[C/T]G AGC ATG GAA (SEQ ID NO:3) at NC_000003.12:g.108579413 position (GRCh38.p2), which manifests itself at the expression level as the mRNA sequence TTC CAT GCT C[G/A]A AAC (SEQ ID NO:4). The "C allele" of the C/T SNP of rs2278911 is the major *CIP2A* allele, and it may also be referred to by the terms "non-risk allele", "non-disease-associated allele", and *"CIP2A* rs2278911 (C)". Accordingly, the "T allele" of the C/T SNP of rs2278911 is the minor *CIP2A* allele, and it may be referred to by the terms "risk allele", "disease-associated allele", *"CIP2A* rs2278911 (T)", and the like.

The change of cytosine for thymine at NC_000017.11:g.48728343 position (GRCh38.p2) in *CIP2A* results in amino acid variation denoted "R229Q", wherein position 229 encodes a glutamine (Q) instead of an arginine (R). Accordingly, the R229Q variant of *CIP2A* may be referred to by the term "risk variant" or "disease-associated variant". As used herein, the terms *"CIP2A* R229Q", *"CIP2A* rs2278911 (T)", and *"CIP2A* T allele" are interchangeable.

As used herein, the term *"HOXB13* T and *CIP2A* T dual carrier", and any equivalent expressions, refer to a subject who carries T alleles of both *HOXB13* and *CIP2A.* Independently from each other, said T alleles may be present in either homozygous or heterozygous form. Thus, said dual carrier may be a subject having a genotype of *HOXB13* TT and *CIP2A* TT, *HOXB13* CT and *CIP2A* TT, *HOXB13* TT and *CIP2A* CT, or *HOXB13* CT and *CIP2A* CT. The expressions *"HOXB13* T and *CIP2A* T", *"HOXB13* T allele and *CIP2A* T allele", *"HOXB13* rs138213197 (T) and *"CIP2A* rs2278911 (T)", *"HOXB13* G84E and *CIP2A* R229Q", and any linguistic variations thereof, are interchangeable and they all refer to the concurrent presence of T allele of *HOXB13* and T allele of *CIP2A.*

Accordingly, the term *"HOXB13* T carrier" refers to a subject who carries at least one *HOXB13* T allele and who may carry either *CIP2A* C or T allele. In other words, said *HOXB13* T carrier may be a subject having a genotype of *HOXB13* TT and *CIP2A* TT, *HOXB13* TT and *CIP2A* CT, *HOXB13* TT and *CIP2A* CC, *HOXB13* CT and *CIP2A* TT, *HOXB13* CT and *CIP2A* CT, or *HOXB13* CT and *CIP2A* CC. The presence of either C or T allele of *CIP2A* may be indicated by the term "CIP2A rs2278911 (C/T)".

The term *"CIP2A* T carrier" refers to a subject who carries at least one *CIP2A* T allele and who may carry either *HOXB13* C or T allele. In other words, said *CIP2A* T carrier may be a subject having a genotype of *HOXB13* TT and *CIP2A* TT, *HOXB13* CT and *CIP2A* TT, *HOXB13* CC and *CIP2A* TT, *HOXB13* TT and *CIP2A* CT, *HOXB13* CT and *CIP2A* CT, or *HOXB13* CC and *CIP2A* CT. The presence of either C or T allele of *HOXB13* may be indicated by the term "rs138213197 (C/T)".

As used herein, the term "non-dual T carrier" refers to a subject who carries neither T allele of *HOXB13* nor T allele of *CIP2A* T; or who carries either T allele of *HOXB13* or T allele of *CIP2A,* but not both. In other words, a non-dual T carrier may have a genotype *HOXB13* CC and *CIP2A* CC; genotype *HOXB13* CT and *CIP2A* CC; genotype *HOXB13* TT and *CIP2A* CC; genotype *HOXB13* CC and *CIP2A* CT; or genotype *HOXB13* CC and *CIP2A* TT.

The expression "the presence of" a specific allele or variant indicates that a subject carrying said allele or variant it at least heterozygous for the same. Thus, the expression "the presence of *HOXB13* rs138213197 (T)", for instance, is interchangeable with the expression "the presence of at least one T allele of *"HOXB13* rs138213197". Both expressions mean that the subject in question may be either heterozygous or homozygous for *HOXB13* rs138213197 (T). Corresponding nomenclature applies for *CIP2A.*

A subject's genetic predisposition to, susceptibility to, or risk of prostate cancer may be expressed as a probability, such as an odds ratio or hazard ratio. As used herein, the term "odds ratio" (OR) refers to the ratio of the frequency of the disease in individuals having a particular marker (allele or polymorphism) to the frequency of the disease in individuals without the marker (allele or polymorphism). Odds ratios are most commonly used in case-control studies but may also be applied in cross-sectional and cohort study designs as is well known in the art.

As used herein, the term "hazard ratio" (HR) refers to the ratio of an instantaneous risk in two experimental arms over the study time period. It represents point estimate at any given point of time, thus not being a cumulative estimate like the odds ratio. Hazard ratios are usually applied in survival analysis as is well known in the art.

Thus, with respect to a disease risk, if the risk estimate (OR or HR) for a particular allele at a SNP position is greater than one, this indicates that an individual with this particular allele has a higher risk for the disease than an individual who has the other allele at the SNP position. In contrast, if the risk estimate (OR or HR) for a particular allele is less than one, this indicates that an individual with this particular allele has a reduced risk for the disease compared with an individual who has the other allele at the SNP position.

As used herein, the term "95% confidence interval" (95% CI) refers to an estimated range of values that one can be 95% certain contains the true mean of the population, the estimated range being calculated from a given set of sample data. Generally, the 95% CI is used to estimate the precision of the OR or HR. A wide CI indicates a low level of precision of the OR or HR, whereas a narrow CI indicates a higher precision of the OR or HR.

As used herein, the terms "increased risk" and "higher risk" are interchangeable and they refer to a probability that is at least about 1.5 times, at least about 1.75, at least about 2 times, at least about 3 times, at least about 4 times, at least about 6 times, at least about 7 times, at least about 8 times, at least about 9 times, at least about 10 times, at least about 15 times, at least about 20 times, at least about 25 times, at least about 30 times, at least about 35 times, or at least about 40 times higher than a corresponding probability in a relevant control. In some embodiments, said relevant control is a subject who does not carry *HOXB13* rs138213197 (T), while in some other embodiments, said relevant control is a non-dual T carrier, such as a subject who carries neither *HOXB13* rs138213197 (T) nor *CIP2A* rs2278911 (T). Those skilled in the art can easily choose the relevant control for each case.

Turning now to the experimental part, the present allele-level studies show that *HOXB13* rs138213197 (T) was associated with significantly increased overall risk of prostate cancer in unselected non-familial cases (OR 8.0; 95% CI 5.0-12.8; p=7.69E⁻²⁵). The association was stronger with clinically detected prostate cancer vs. controls (OR 8.6; 95% CI 5.4-13.8; p=3.1x10⁻¹⁹) than with screen-detected prostate cancer vs. controls (OR 5.2; 95% CI 2.8-9.9; p=3.4x10⁻⁷). This indicates that T carriers of *HOXB13* rs138213197 have higher risk for clinically relevant prostate cancer than for clinically non-relevant cancer. Moreover, T carriers of *HOXB13* rs138213197 showed earlier than an average onset of prostate cancer among Caucasian men (HR 1.9; 95% CI, 1.6-2.2; p=6.07E-16), and significantly increased risk of progression into CRPC as compared with non-T-carriers (OR 10.8; 95% CI 5.8-19.9; p=3.1x10⁻¹⁴). Taken together, these results warrant the use of *HOXB13* rs138213197 (T) as a predictive, diagnostic, and/or prognostic marker of prostate cancer, especially clinically relevant prostate cancer including aggressive prostate cancer and particularly CRPC.

As also disclosed in the experimental part, the risk of prostate cancer was especially high in *HOXB13* rs138213197 T carriers with prior BPH diagnosis (OR 12.6; 95% CI, 2.8-56.8; p=0.001). This is an important result because, generally, BPH is not considered as a precancerous condition. Accordingly, in some embodiments it is advantageous to employ *HOXB13* rs138213197 (T) for determining the risk of prostate cancer, diagnosing prostate cancer and/or prognosing prostate cancer in a subject having earlier diagnosis of BPH.

The experimental part also discloses a statistically significant association between the presence of *HOXB13* rs138213197 (T) and a high PSA level (>20 ng/mL) at diagnosis (OR 1.5; 95% CI, 1.1-2.3; p=0.012). Accordingly, in some embodiments, it is preferable to use *HOXB13* rs138213197 (T) determinations in combination with PSA measurements in determining a subject's risk of prostate cancer, diagnosing prostate cancer and/or prognosing prostate cancer. Concurrent presence of *HOXB13* rs138213197 (T) and high PSA level (>20 ng/mL) indicate the presence or risk of having or developing clinically relevant prostate cancer.

Furthermore, the experimental part shows that dual carriers of *HOXB13* rs138213197 (T) and *CIP2A* rs2278911 (T) have remarkably higher odds of developing prostate cancer than non-dual T carriers (OR 21.1; 95% CI 5.2-87.5; p=0.000024). Again, the association was stronger with clinically detected prostate cancer vs. controls (OR, 23.4; 95% CI 5.7-96.8, p=0.000013) than with screen-detected prostate cancer vs. control (OR 10.7; 95% CI 2.0- 58.8; p=0.006). This indicates that dual T carriers are more likely to have or develop clinically relevant prostate cancer than clinically non-relevant cancer. These results warrant the use of *HOXB13* rs138213197 (T) and *CIP2A* rs2278911 (T) as a predictive, diagnostic, or prognostic markers of prostate cancer, especially clinically relevant prostate cancer.

Moreover, dual T carriers of *HOXB13* rs138213197 and *CIP2A* rs2278911 showed earlier than an average onset of prostate cancer among Caucasian men (HR 2.1; 95% CI, 1.6-2.8; p=4.52E-7), warranting closer monitoring of dual carriers for the development or onset of prostate cancer.

The experimental part also demonstrates that dual T carriers of *HOXB13* rs138213197 and *CIP2A* rs2278911 have higher odds of developing prostate cancer with a high Gleason score (≥ 8) than non-dual T carriers (OR 2.3; 95% CI 1.1-4.8; p=0.025). Moreover, dual T carriers of *HOXB13* rs138213197 and *CIP2A* rs2278911 have 36.6 times higher risk of developing castration-resistant prostate cancer as compared with non-dual T carriers (OR 36.6; 95% CI 7.5-168.5; p=0.000007). Both above results demonstrate that dual T carriers of *HOXB13* rs138213197 and *CIP2A* rs2278911 have higher odds of developing aggressive prostate cancer as compared to non-dual T carriers. Naturally, having aggressive prostate cancer predicts poorer prognosis. Indeed, the experimental part shows that dual carriers of *HOXB13* and *CIP2A* T alleles have significantly worse survival rate and die earlier of prostate cancer than non-dual T carriers (HR 3.9; 95% CI 91.6-94.5; p=0.048). These results further attest the value of *HOXB13* rs138213197 (T) and *CIP2A* rs2278911 (T) as a predictive, diagnostic, or prognostic markers of prostate cancer, especially aggressive prostate cancer.

The herein-disclosed risk effects of *HOXB13* rs138213197 (T), particularly in combination with *CIP2A* rs2278911 (T), in various aspects of clinically relevant prostate cancer are remarkably higher than those of known prostate cancer-associated SNPs. Generally, the ORs of the known SNPs range between 1.04 and 2.9.

In one aspect, the present invention provides a method which comprises providing a sample obtained from a subject; and testing said sample for *HOXB13* rs138213197 and *CIP2A* rs2278911. More specifically, said method may be a method of analysing a subject for prostate cancer-associated markers, a clinical risk determination method, or a method of predicting prostate cancer, determining risk of prostate cancer (including determining risk of having or developing prostate cancer), determining genetic predisposition or susceptibility to prostate cancer, determining the presence of prostate cancer, diagnosing prostate cancer and/or prognosing prostate cancer. In other words, said method may, in some embodiments, be phrased as a method of predicting, diagnosing and/or prognosing prostate cancer. In the present method, and in any embodiments thereof, the presence of *HOXB13* rs138213197 (T), preferably in combination with *CIP2A* rs2278911 (T), indicates that said subject is at increased risk of having or developing prostate cancer. On the other hand, the absence of *HOXB13* rs138213197 (T) either alone or in combination with the absence of *CIP2A* rs2278911 (T) indicates that said subject is not at increased risk of having or developing prostate cancer.

In some embodiments of the present method, the presence of *HOXB13* rs138213197 (T), irrespective of the subject's *CIP2A* carrier status, i.e. the presence of *CIP2A* rs2278911 (C/T), indicates increased risk of prostate cancer, presence of clinically-relevant prostate cancer, presence of aggressive prostate cancer, especially CRPC, or increased risk of progression into clinically relevant or aggressive prostate cancer, especially CRPC. In some further embodiments said increased risk indicates earlier than an average onset of prostate cancer. In some still further embodiments, the subject to be analysed for the risk of prostate cancer, diagnosed for the presence of prostate cancer, or analysed for the risk of progression of prostate cancer, has an earlier diagnosis of BPH. In one embodiment of the present method, the subject to be analysed for the risk of prostate cancer, diagnosed for the presence of prostate cancer, or analysed for the risk of progression of prostate cancer, belongs to a family with a high incidence of prostate cancer.

In some other embodiments of the present method, the presence of *HOXB13* rs138213197 (T) and of *CIP2A* rs2278911 (T) indicates the presence of, a remarkably increased risk of having or developing, or a remarkable susceptibility or genetic predisposition to prostate cancer, especially clinically relevant prostate cancer, aggressive prostate cancer, or CRPC; or increased risk of progression into aggressive prostate cancer, especially CRPC. In some further embodiments said increased risk indicates earlier than an average onset of prostate cancer.

In some further embodiments, the herein-disclosed risk effects are higher in subjects who are homozygous for one or both of the present risk alleles than in subjects who are heterozygous for both of the risk alleles. In other words, the risk effects increase with the increasing number of disease-associated T alleles. A subject who is homozygous for both *HOXB13* rs138213197 (T) and *CIP2A* rs2278911 (T) has four (i.e. the maximum number) disease-associated T alleles.

In some still other embodiments, determining susceptibility or predisposition of a subject to prostate cancer means determining the risk of said subject of getting prostate cancer prior to any diagnosis of prostate cancer. Thus, in some embodiments, the present method is to be carried out prior to any prostate cancer diagnosis. In such embodiments, the presence of at least one T allele of the *HOXB13* rs138213197, especially together with the presence of at least one T allele of the *CIP2A* rs2278911, is indicative of increased risk of said subject of getting prostate cancer. Accordingly, detecting the presence of *HOXB13* rs138213197 (T), and especially detecting the concurrent presence of *HOXB13* rs138213197 (T) and of *CIP2A* rs2278911 (T), would justify scheduled medical examinations or more frequent screening in subjects in whom prostate cancer is not yet diagnosed, but in whom an increased risk of prostate cancer, especially aggressive prostate cancer, is identified by any one of methods disclosed herein.

Thus, the present method may be used for categorizing subjects into a high-risk group or a low-risk group on the basis of their *HOXB13* and *CIP2A* genotype, such that subjects who are carriers of *HOXB13* T allele, and especially dual carriers of *HOXB13* and *CIP2A* T alleles, are assigned to the high-risk group, while non-carriers of *HOXB13* T allele, and especially non-dual carriers of *HOXB13* and *CIP2A* T alleles, are assigned to the low-risk group. In some embodiments, such grouping may be used for stratifying high-risk subjects not diagnosed with prostate cancer for active monitoring for the onset of prostate cancer, for early intervention, for preventive therapies, or for therapeutic trials for the same with the aim of preventing or delaying the appearance of prostate cancer or aggressive prostate cancer.

Accordingly, the present method and herein-disclosed prostate cancer risk determinations may be incorporated into a population screening protocol to identify subjects predisposed or susceptible to prostate cancer prior to onset of the disease and/or prior to increase in the PSA levels. This would enable active surveillance for the onset of prostate cancer, e.g. by more frequent PSA testing, biparametric magnetic resonance imaging (MRI), and/or digital rectal examination (DRE). Such screening would also enable the subjects to take appropriate steps aiming to prevent, delay, or reduce the severity of prostate cancer. Such appropriate steps are well known in the art and include choosing a healthy diet, reducing weight, and exercising. Moreover, early detection of prostate cancer would allow treating the disease when chances of cure are the highest.

In other embodiments, determining the susceptibility, predisposition, or risk of a subject to aggressive prostate cancer is performed either prior to, at the time, or after the diagnosis of prostate cancer, wherein the presence of at least one T allele of the *HOXB13* rs138213197, especially in combination with the presence of at least one T allele of the *CIP2A* rs2278911, is indicative of an increased risk of said subject of having aggressive prostate cancer or having prostate cancer later progressing to aggressive prostate cancer.

In some further embodiments, testing for *HOXB13* rs138213197 and *CIP2A* rs2278911 is carried out in a sample obtained from a subject already diagnosed with prostate cancer. In such embodiments, the presence of *HOXB13* rs138213197 (T), and especially the simultaneous presence of *HOXB13* rs138213197 (T) and *CIP2A* rs2278911 (T), indicates an increased risk for the presence of, or progression to an aggressive form of prostate cancer.

The present risk determinations warrant active surveillance of high-risk subjects for the progression of prostate cancer, and provide substantial help in clinical decision making in choosing appropriate treatment procedures and in focusing resources upon those groups of subjects at highest need. For example, concurrent presence of *HOXB13* and *CIP2A* T alleles in a subject having prostate cancer diagnosis indicates that it might be beneficial to use a more aggressive treatment strategy, such as radiotherapy or prostatectomy, instead of a less aggressive treatment strategy. On the other hand, the absence of *HOXB13* rs138213197 (T), either alone or in combination with the absence of *CIP2A* rs2278911 (T), indicates that less aggressive treatment would be sufficient.

Thus, the above-disclosed risk categorization of subjects on the basis of their *HOXB13* and *CIP2A* genotype may be used for stratifying subjects into a high-risk group with aggressive prostate cancer or into a low-risk group with indolent or clinically insignificant prostate cancer. As used herein, the terms "indolent prostate cancer" and "clinically insignificant prostate cancer" are interchangeable and mean a prostate cancer with limited clinical significance, including a localized and/or slowly growing prostate cancer, a prostate cancer that is unlikely to progress to an advanced or lethal disease if left untreated, and/or a prostate cancer not related to significant morbidity or mortality of the disease. Synonyms for clinically insignificant prostate cancer include, among others, low-grade, small-volume, low-volume, minimal, organ-confined, minute and micro-focal prostate cancer.

In some embodiments, the present methods are used for the stratification of subjects diagnosed with prostate cancer in a high-risk subgroup with a high probability of having or developing aggressive prostate cancer and a low-risk subgroup with a high probability of having an indolent or clinically insignificant prostate cancer. In some further embodiments, subjects stratified to the high-risk and low-risk subgroups preferentially receive differential personalized therapy for prostate cancer based on their different estimated prostate-cancer related clinical endpoints. The subjects in the high-risk group are more likely to benefit from, and thus more likely to be treated by, radical prostatectomy or radiotherapy than subjects in the low-risk group.

In some even further embodiments, subjects with a high estimated risk of developing prostate cancer or aggressive prostate based on said subject's dual carrier status of *HOXB13* and *CIP2A* T alleles are selected for focused clinical surveillance, which may include e.g. medical examinations performed more often, or monitoring of cancer or prostate cancer-associated biomarker(s), with the aim of closer monitoring and/or earlier therapy of prostate cancer upon appearance as compared to non-dual carriers of the *HOXB13* and *CIP2A* T alleles.

The present invention provides advantages not only for individual patient care but also for better selection and stratification of patients for clinical trials. For example, patients with significantly increased risk of aggressive forms or prostate cancer could be selected to clinical studies with the aim of developing efficient new therapeutic tools such as new medical procedures or drugs.

Furthermore, the present invention and its various embodiments may be employed in pharmacogenomic studies, which aim at analysing and predicting a response to a prostate cancer treatment or a drug. Accordingly, testing a subject for *HOXB13* rs138213197 and *CIP2A* rs2278911 may be carried out in any of the following cohorts: a cohort that responds favourably to a treatment regimen, a cohort that does not respond favourable to a treatment regimen, a cohort that responds significantly to a treatment regimen, a cohort that does not respond significantly to a treatment regimen, and a cohort that responds adversely to a treatment regimen (e.g. exhibits one or more side effects). These cohorts are provided as examples only, and any other cohorts or sub-cohorts may be analysed by the present method. Thus, a subject may be genotyped or tested for *HOXB13* rs138213197 and *CIP2A* rs2278911 to predict whether he will respond favourably or significantly to a treatment regiment, not respond favourably or significantly to a treatment regimen, or respond adversely to a treatment regimen. If a subject is likely to respond positively to a prostate cancer treatment with a drug, the drug may be administered to said subject. On the other hand, if a subject is likely to respond negatively (i.e. not exhibit positive effects, or exhibit adverse side effects) to prostate cancer treatment with a drug, an alternative course of treatment may be applied.

Moreover, the present invention and its various embodiments also give grounds for targeted molecular studies aiming to solve the biological background and rationale behind the synergistic effect of the *HOXB13* and *CIP2A* T variants.

Our own recent functional studies evaluating the biological interaction between HOXB13 and CIP2A strengthen our observations of synergism in prostate cancer. Using Chromatin ImmunoPrecipitation (ChIP) with antibodies against HOXB13, coupled to massively parallel sequencing (ChIP-seq), strong chromatin binding of HOXB13 at CIP2A locus was observed both in prostate cancer cell lines and tumor tissue samples (unpublished data). HOXB13 functionally regulates the expression of CIP2A, showed by short hairpin RNA (shRNA)-mediated knockdown of HOXB13 in prostate cancer cell line. HOXB13 knockdown leads to markedly decreased level of CIP2A, suggesting that CIP2A is a direct regulatory target of HOXB13.

In the present method, and in any embodiments thereof, results from the genetic risk determinations may be combined with other test results indicative of prostate cancer, such as results of testing for one or more prostate cancer-associated biomarkers, biparametric MRI, or DRE, which were previously, concurrently, or subsequently gathered with respect to the genetic predisposition testing. Alternatively or in addition, results from the present genetic risk determination may be analysed in combination with other risk factors or clinical variables, such as age, family history, previous prostate biopsy, previous BPH, the results of a prostate exam (e.g. by biparametric MRI and/DRE) and/or body mass index. In some further embodiments, the combination of the genetic risk determination results with other test results and/or risk factors or relevant clinical variables can be indicative or probative of prostate cancer, especially aggressive prostate cancer, and the combination may be utilized as an embodiment of any one of the herein-disclosed methods.

In some preferred embodiments, the determination of *HOXB13* rs138213197 and *CIP2A* rs2278911 is preceded by, combined with or followed by the measurement of at least one prostate cancer-associated biomarker with the purpose of increasing the predictive, diagnostic and/or prognostic value of the present method. Non-limiting examples of prostate cancer-associated biomarkers suitable for being tested from blood include total prostate specific antigen (tPSA), free prostate specific antigen (fPSA), intact prostate specific antigen (iPSA), proPSA, human kallikrein 2 (hK2), microseminoprotein-beta (MSMB), and macrophage inhibitory cytokine-1 (MIC1). In some embodiments, a combination or ratio of prostate cancer-associated biomarkers is employed. Examples of such combinations or ratios include the ratio of iPSA and tPSA either alone or in combination with the determination of hK2, the ratio of fPSA to tPSA, and the Prostate Health Index (PHI). Non-limiting examples of prostate cancer-associated biomarkers suitable for being tested from urine include prostate cancer antigen 3 (PCA3) and TMPRSS2-ERG gene fusion product, while ERG is a non-limiting example of prostate cancer-associated tissue markers that may be detected by immunohisto-chemistry. The present method may also be used in combination with testing for any further, e.g. future or emerging, prostate cancer-associated biomarker including, but not limited to, other genetic polymorphisms in *HOBX13* and/or *CIP2A,* genetic polymorphisms in other genes such as PTEN, RAF, BRAF, SPOP, EZH2, and Spink1, as well as gene regulating factors (e.g. eQTLs, transcription factors, enhancers, methylation marks). Those skilled in the art know how to measure or test for these prostate cancer-associated biomarkers, and understands that also any biologically activated or inactivated derivatives or genetic variants of any the above-mentioned biomarkers may be employed, if appropriate.

The present invention and its various embodiments provide an easily applicable, simple and rapid tool for predictive prognostics and diagnostics of prostate cancer because it targets germline mutations and can hence directly be applied on any sample type containing somatic cells of the subject, not restricting the method to samples obtained by biopsy. Suitable sample types include but are not limited to whole blood, plasma, serum, saliva, urine, tissue and/or smear samples taken e.g. by swabbing the mucous membranes such as the oral mucosa.

The presence or absence of *HOXB13* and *CIP2A* alleles of interest, preferably the T alleles, in a sample obtained from a subject can be determined by any available or future means suitable for this purpose and at different molecular levels. The determination of the T alleles can be performed at the genetic level (i.e., gene-level) by analysing DNA, or at the expression-level by analysing RNA or protein. The presence or absence of the *HOXB13* and *CIP2A* T alleles may be determined independently from each other, i.e. either at the same or different molecular levels and/or by the same or methods or techniques. Some methods may allow multiplexed simultaneous testing for *HOXB13* rs138213197 and *CIP2A* rs2278911. Preferably, the same method is used for analysing a sample for *HOXB13* rs138213197 and *CIP2A* rs2278911.

Furthermore, a subject's carrier status regarding one or both of the present risk alleles (i.e., *HOXB13* T allele, *CIP2A* T allele, or both) may be assessed directly by determining the presence or absence of said risk alleles in a sample taken from said subject. Alternatively, the occurrence of one or both of the risk alleles may be assessed indirectly by determining the presence or absence of a corresponding non-risk allele in a sample taken from said subject. In other words, absence of a non-risk allele indicates the presence of a corresponding risk allele in homozygous form, whereas, depending on the method used, presence of a non-risk allele may indicate either absence of a corresponding risk allele or that the subject is heterozygous for the risk and non-risk alleles. Generally, determining the presence or absence of the allele or variant of interest may be referred to as "testing a sample for *HOXB13* rs138213197 and *CIP2A* rs2278911".

Accordingly, in some embodiments, testing for *HOXB13* rs138213197 and *CIP2A* rs2278911 may be carried out using a method which simply indicates whether or not the *HOXB13* and/or *CIP2A* allele of interest occurs in a subject's genome. In other words, if the allele of interest is present, the method does not tell whether the subject is heterozygous or homozygous for the presence of said allele. In some other embodiments, testing for *HOXB13* rs138213197 and *CIP2A* rs2278911may be carried out by a method which is able to detect both risk and non-risk alleles. Using such a method, one can determine both the presence of a risk allele in a subject's genome and whether the subject is homozygous or heterozygous for the risk allele.

Various methods for determining the presence or absence of the *HOXB13* and *CIP2A* alleles of interest, preferably the risk alleles, are available and familiar to those skilled in the art. Accordingly, testing for *HOXB13* rs138213197 and *CIP2A* rs2278911may be performed, for instance, by nucleic acid sequencing. To this end, primers flanking the SNP are selected and used to amplify the region comprising the SNP. The amplified region is then sequenced using any known sequencing technique to determine which *HOXB13* and/or *CIP2A* SNP allele(s) is (are) present in the sample. Non-limiting examples of suitable of nucleic acid sequencing methods for use in the present invention include Sanger sequencing, NGS-based applications, single-cell sequencing, and minisequencing.

Presence or absence of a particular allele may also be detected using an allele-specific amplification reaction, wherein allele-specific primers support primer extension only if the targeted allele is present. Alternatively, testing for *HOXB13* rs138213197 and *CIP2A* rs2278911 may be carried out by primer extension assays using primers which hybridize to the nucleotides immediately upstream of the SNP. A DNA polymerase then extends the hybridized primer by incorporating a detectable nucleotide that is complementary to the SNP. Typically, detectable nucleotides comprise a label capable of producing, either directly or indirectly, a detectable signal. Similarly, any available or future nucleic acid amplification assays, including but not limited to polymerase chain reaction (PCR), ligase chain reaction (LCR), loop-mediated amplification (LAMP), helicase-dependent amplification (HDA), nicking enzyme amplification reaction (NEAR), transcription-mediated amplification (TMA), nucleic acid sequence based amplification (NASBA), strand displacement amplification (SDA) and various other temperature-dependent or isothermal amplification methods may be employed in testing a sample for *HOXB13* rs138213197 and *CIP2A* rs2278911.

In further embodiments, determining the presence or absence of the *HOXB13* and *CIP2A* alleles of interest may involve differential susceptibility of the polymorphic site to restriction enzymes. For this purpose, PCR may be used to amplify the region comprising the SNP. The amplified PCR product is then subjected to a restriction enzyme digestion followed by electrophoretical analysis of the restriction product. If a specific SNP allele is associated with a specific restriction site, digestion of that site is indicative of the presence of that particular SNP.

Many further types of allelic discrimination assays are known in the art. A subject's *HOXB13* rs138213197 and/or *CIP2A* rs2278911 genotype may also be determined using an oligonucleotide probe specific to either C or T allele of *HOXB13* or *CIP2A.* In some embodiments, a region comprising the SNP is amplified prior to, or concurrent with, carrying out a hybridization reaction with the probe. Hybridization of the probe to the amplification product under stringent hybridization conditions indicates that the amplified region contains that particular allele of *HOXB13* or *CIP2A* for which the probe is specific for.

Further methods suitable for determining the presence or absence of *HOXB13* and *CIP2A* alleles of interest at the nucleic acid or genetic level include, but are not limited to, RNase protection assays, molecular beacon-based oligonucleotide binding assays, melting curve analysis combined with oligonucleotide probes and/or intercalating labels, gel electrophoresis analysis, Southern blotting, microarrays such as DNA microarrays and RNA microarrays, direct probing, and signal accumulation assays with or without amplification of the target nucleic acids.

As set forth above, determining a subject's carrier status for *HOXB13* rs138213197 and/or *CIP2A* rs2278911, i.e. determining the presence or absence of *HOXB13* and *CIP2A* alleles of interest, may be carried out at protein level. The presence of *HOXB* G84E or *CIP2A* R229Q is a direct indication of the presence of the corresponding risk alleles (T alleles). Non-limiting examples of methods suitable for protein-level analysis include mass spectrometry, immunoassays, immunohistochemistry, and other protein-binding assays. In some specific embodiments, one or more antibodies or antibody fragments specific for a protein or polypeptide encoded by a particular polymorphic form of *HOXB13* or *CIP2A* may be used for the determination. Binding bodies alternative to antibodies include but are not limited to aptamers, receptors and biologically interacting proteins, such as up- or down regulating targets identified in relevant biological signalling pathways.

Any available or future mRNA detection technique may be applied for determining the presence or absence of *HOXB13* and *CIP2A* alleles of interest at the expression level. Non-limiting examples of methods suitable for mRNA-level analysis include reverse transcriptase PCR (RT-PCR), RNA microarrays, and mass spectrometry.

The present invention also provides a kit for use in the present method and any embodiments thereof. The kit comprises a first binding body for testing said sample for *HOXB13* rs138213197 and a second binding body for testing said sample for *CIP2A* rs2278911. Said first binding body may be specific for determining the presence or absence of either *HOXB13* rs138213197 (T) or *HOXB13* rs138213197 (C). Independently from the specificity of the first binding body, said second binding body may be specific for determining the presence or absence of either *CIP2A* rs2278911 (T) or *CIP2A* rs2278911 (C). In some embodiments, the kit may even comprise a first binding body for specifically determining the presence or absence of *HOXB13* rs138213197 (T) and a different first binding body for specifically determining the presence or absence of *HOXB13* rs138213197 (C) and/or a second binding body for specifically determining the presence or absence of *CIP2A* rs2278911 (T) and a different second binding body for specifically determining the presence or absence of *CIP2A* rs2278911 (C). Such embodiments are typically employed for determining whether the sample to be analyzed with the kit is homozygous or heterozygous for *HOXB13* rs138213197 and/or *CIP2A* rs2278911.

Depending on the detection technique and the level of detection (i.e. detection at gene-level, mRNA-level, or protein-level) to be employed, those skilled in the art can easily choose appropriate first and second binding bodies to be included in the kit, for determining the presence or absence of *HOXB13* rs138213197 (T), *HOXB13* rs138213197 (C), *CIP2A* rs2278911 (T) and/or *CIP2A* rs2278911 (C). For example, said first and second binding bodies may be selected, independently from each other, from the group consisting of nucleic acid primers, nucleic acid probes, aptamers, antibodies or antibody fragments, peptides, receptors, and enzymes, including restriction enzymes. Those skilled in the art can readily choose appropriate binding bodies depending on the detection technique to be employed.

For detection, binding bodies may be conjugated or otherwise associated with a detectable label selected from the group including, but not limited to, optical agents such as luminescent or fluorescent labels including a variety of organic and/or inorganic small molecules and a variety of fluorescent proteins and derivatives thereof, phosphorescent labels, chemiluminescent labels, and chromogenic labels; radioactive labels such as radionuclides that emit gamma rays, positrons, beta or alpha particles, or X-rays; and enzymes such as alkaline phosphatase (AP), luciferase, or (horseradish) hydrogen peroxidase (HRP). Said association can be direct, e.g. through a covalent bond, or indirect, e.g. via another binding agent, chelator, or linker. Also, the label may be directly detectable or indirectly detectable through a detectably labelled secondary binding body. Detectable labels and techniques for conjugating or otherwise associating said detectable labels to binding bodies are well known in the art. Furthermore, those skilled in the art can readily choose appropriate detectable labels depending on the type of binding bodies to be employed.

It will be obvious to a person skilled in the art that, as technology advances, the inventive concept can be implemented in various ways. The invention and its embodiments are not limited to the examples described below but may vary within the scope of the claims.

### EXAMPLES

### Example 1. Sequencing and SNP genotyping of study subjects

### Study subjects

The study was performed in Finland and all cancer cases and controls involved and genotyped in this study were of Caucasian origin. Written informed consent was obtained from each study subject. The study protocol was approved by the research ethics committee at Pirkanmaa Hospital District (Tampere, Finland) and by the National Supervisory Authority for Welfare and Health (VALVI-RA).

For *HOXB13* mutation 2669 and for *CIP2A* variant 2738 unselected non-familial eligible prostate cancer (PrCa) cases were analyzed, respectively. The genotyping was partly carried out by the PRACTICAL (PRostate cancer AssoCiation group To Investigate Cancer Associated aLterations in the genome) consortium. Of cases, 2281 were clinically diagnosed cases from the Pirkanmaa Hospital District, confirmed from medical records. Another set of subjects consisted of 457 Finnish screen detected cancer cases recruited by the Finnish arm of The European Randomized Study of Screening for Prostate Cancer (ERSPC). This study was initiated in the mid-1990s to evaluate the effect of prostate-specific antigen (PSA) screening on death rates from PrCa (Schröder et al., N Engl J Med. 2009;360:1320-8). Of the patients, 106 were young-onset cases.

PrCa control subjects (*HOXB13* G84E, n=2423; *CIP2A* R229Q, n=2427), belonging to the screening trial control group, were derived from the Finnish arm of the ERSPC (Schröder *et al., Ibid*)*.* Control subjects were population-matched healthy individuals of ages between 70 and 86 years who had undergone PSA screening. Their disease status is annually evaluated from the records of the Finnish Cancer Registry.

### Clinical and pathological sub-classification of PrCa patients

Clinically detected PrCa cases (83.3%) dominated the sample set compared to a smaller proportion of screen-detected patients (16.7%). Notably, 27.4% of all cases developed either PSA progression, local or distant progression, and 10.5% emerged castration resistance after androgen deprivation therapy. From 1126 deceased PrCa patients 26.4% died of PrCa, which is 10.9% of the whole PrCa population studied. In addition, germline DNA was available from altogether 590 clinically and pathologically defined BPH from the Urology Outpatient Clinic in Tampere University Hospital (Tampere, Finland). From these, 198 (33.6 % of BPH patients) developed PrCa later in life (7.23% of all PrCa cases). Clinical characteristics of genotyped PrCa patients are summarized in Table 1.

**Table 1. Clinical characteristics of prostate cancer patients**

| **Total PrCa sample size** | **n=2738 (%)** |
|---|---|
| Average age at onset (years) | 68.1 |
| Average age at progression (years) | 72.3 |
| Average age at death (years) | 78.4 |
| Other cancer present | 543 (19.8) |
| Castration resistance prostate cancer (CRPC) | 287 (10.5) |
| Benign prostatic hyperplasia (BPH) cases | 198 (7.23) |

| **Age at diagnosis (years)** | |
|---|---|
| <=55, young onset | 106 (3.90) |
| >55 | 2632 (96.1) |

| **Diagnostic PSA level, ng/mL** | |
|---|---|
| Low, ≤20 | 2099 (76.7) |
| High, >20 | 484 (17.7) |
| Missing data | 155 (5.7) |

| **Gleason score** | |
|---|---|
| Low, ≤6 | 1320 (48.2) |
| High, ≥8 | 368 (13.4) |
| Intermediate, =7 | 685 (25.0) |
| Missing data | 365 (13.3) |

| **T stage** | |
|---|---|
| T0 | 4 (0.10) |
| T1 | 1105 (40.4) |
| T2 | 972 (35.5) |
| T3 | 443 (16.2) |
| T4 | 97 (3.50) |
| Unknown | 9 (0.30) |
| Missing data | 108 (3.90) |

| **N stage** | |
|---|---|
| N1 | 14 (0.5) |
| NO | 56 (2.00) |
| Unknown | 2560 (93.5) |
| Missing data | 108 (3.95) |

| **M stage** | |
|---|---|
| M1 | 191 (6.98) |
| M0 | 1170 (42.7) |
| Unknown | 1269 (46.3) |
| Missing data | 108 (3.94) |

| **WHO grade** | |
|---|---|
| Low grade, WHO 1 and 2 | 1850 (67.6) |
| High grade, WHO 3 | 287 (10.5) |
| Missing data | 601 (22.0) |

| **Progression** | |
|---|---|
| PSA | 726 (96.9) |
| Local | 11 (1.50) |
| Distant | 12 (1.60) |

| **Age at progression** | |
|---|---|
| <=55 | 9 (1.20) |
| >55 | 726 (96.9) |
| Missing data | 14 (1.871 |

| **Vital status** | |
|---|---|
| Dead | 1126 (41.1) |
| Alive | 1612 (58.9) |

| **Cause of death** | |
|---|---|
| PrCa | 297 (26.4) |
| Other reason, overall death | 829 73.6 |

| **Primary treatment** | |
|---|---|
| Prostatectomy | 754 (27.5) |
| Radiotherapy | 399 (14.6) |
| Hormonal therapy | 805 (29.4) |
| Active surveillance | 129 (4.71) |
| Brachytherapy | 83 (3.03) |
| Cystectomy | 12 (0.44) |
| Missing data | 556 (20.3) |

### SNP genotyping and sequencing

Germline blood DNA was collected and isolated according to standard methods, and genotyped for *HOXB13* G84E (rs138213197) using a Custom Taq-Man SNP assay (Applied Biosystems/Life Technologies) and for *CIP2A* R229Q (rs2278911) using custom Illumina iSelect SNP genotyping array platform, designed as part of the Collaborative Oncological Gene-Environment Study (COGS), according to the manufacturer's instructions. BPH samples for *HOXB13* G84E were genotyped by the Technology Centre, Institute for Molecular Medicine Finland (FIMM), University of Helsinki (Helsinki, Finland) using the MassARRAY iPLEX platform (Sequenom, Inc.). Duplicate test samples and four negative controls were included in each 384-well plate. Concordance for the control samples was >99%. In selected set of PrCa samples the studied variants were confirmed through standard Sanger sequencing using ABI PRISM BigDye Termination Cycle Sequencing Ready Reaction Kit (Applied Biosystems/Life Technologies).

### Example 2. Risk of prostate cancer

The *Hardy-Weinberg equilibrium equation* was used to determine whether the proportion of each genotype obtained was in agreement with the expected values as calculated from the allele frequencies.

Statistical analyses were performed with IBM SPSS version 22 (SPSS Inc, Chicago, USA) unless otherwise specified. As the number of parameters was relatively small compared to the number of subjects *unconditional logistic regression analyses* was used to measure the association between *HOXB13* and *CIP2A* variants and the risk of PrCa by estimation of odds ratio (OR) and its 95% confidence interval (CI). P-values were 2-sided and p<0.05 was considered to indicate a statistically significant result.

According to the results, the *HOXB13* G84E and *CIP2A* R229Q mutations were in Hardy-Weinberg equilibrium in both cases (p=0.097 and p=0.739) and controls (p=0.838 and p=0.498), respectively. The overall minor allele frequency for G84E variant was 1.8%, and for R229Q was 13.8% in the entire sample set. The G84E mutation was detected in 187 subjects, of which 167 were patients with prostate cancer (carrier frequency 6.24%) and 20 were healthy controls (carrier frequency 0.80%). The carrier frequency of R229Q variant was 24.7% (n=675) in cases and 26.5% (n=642) in controls (Table 2.).

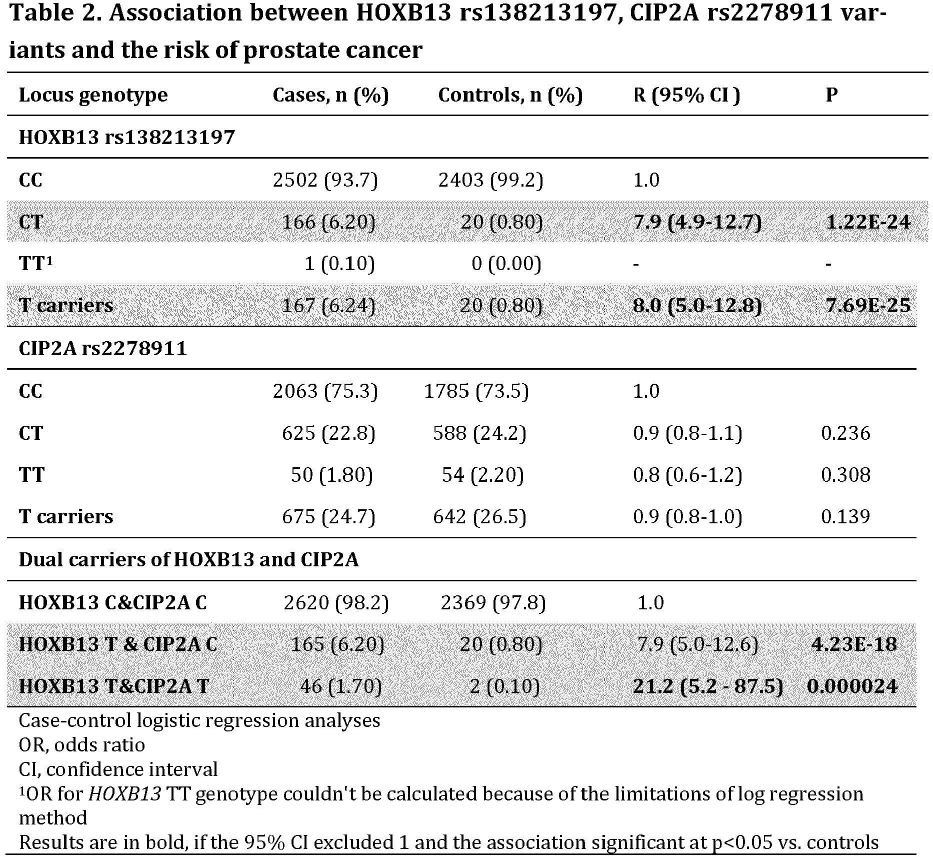

*HOXB13* G84E T allele was associated with 8.0-fold increased risk of PrCa (95% CI 5.0-12.8, p=7.69E-25) in unselected non-familial cases in analyzed Finnish samples.

Significant result was seen for *HOXB13* T and *CIP2A* T dual carriers as they showed 21.1 times increased risk for PrCa (95% CI 5.2-87.5; p=0.000024).

### Example 3. Dissection of the effect of HOXB13 G84E and CIP2A R229Q variants on prostate cancer risk

To evaluate the relative effects of *HOXB13* and *CIP2A* variants on PrCa development *binary stepwise logistic-regression with backward elimination method was* used as described by Cordell et al. (Am J Hum Genet. 2002; 70:124-41.37). By fitting statistical models with main effects, a test with few degrees of freedom that is likely to be powerful for detecting primary etiological determinants was employed. This approach is applicable to case-control studies modelled via unconditional logistic regression. By testing two polymorphisms, a small number of degrees of freedom will be present.

In other words, the effects of *HOXB13* and *CIP2A* variants on PrCa risk were tested with three selection methods (enter, forward and backward) of binary stepwise logistic-regression. The usually preferred backward elimination method gave the most significant result. This procedure was started with all candidate variants, and tested if the deletion of the variant improves the model the most by being deleted, and repeating this process until no further improvement is possible. All of the genetic variants, HOXB13 T carrier, CIP2A T carrier and dual minor allele carrier status (TT), were included in the initial model and a backward stepwise regression selection procedure was used, with an exit p-value of 0.05, to create the final model. Removing *HOXB13* T carriers deteriorated the model most, change in -2 log likelihood was 76.365 points, the significance of change was p=1.3681E-28. Additionally, removing of dual T carriers deteriorated the model as well, but with lower effect (change in -2 Log Likelihood is 4.330 points, P=0.037).

When using all the predictor genetic variants as a group (ENTER method) the Nagelkerke R Square test revealed that 3.3% of the variability in PrCa risk can be accounted for by all of the used predictors together in the Finnish patients. With this method the *HOXB13* T carriers showed significant risk for PrCa (OR 6.268; 95% CI, 3.804-10.327; p=5.8567E-13); but none of other tested variables.

In this way, regression analysis confirmed that *HOXB13* variant has the main effect in PrCa development of all variants used in analyses model.

### Example 4. Time to prostate cancer development

Time to event analyses (carriers vs. non-carriers) were carried out by *Cox regression method.* Results are shown in Table 3 below.

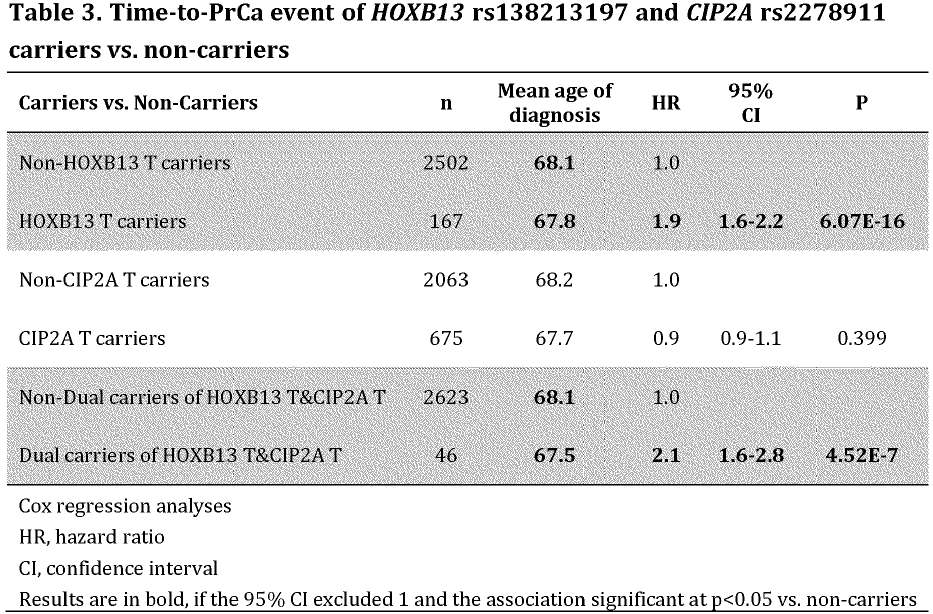

The results showed significant risk for earlier PrCa development in *HOXB13* T allele carriers (HR 1.9; 95% CI, 1.6-2.2; p=6.07E-16), whereas a synergistic effect in dual carriers of *HOXB13* T and *CIP2A* T alleles was shown not only in increasing susceptibility, but also in earlier development of the disease (HR 2.1; 95% CI, 1.6-2.8; p=4.52E-7).

### Example 5. Correlation with clinical features

*Binary logistic regression analyses* were conducted to evaluate the impact of the mutations on tumour phenotype and selected clinical features, including PSA at diagnosis, Gleason-score, general progression, clinical and screen detection, CRPC development, BPH status and PrCa development, general and disease specific death.

Statistically significant associations of *HOXB13* G84E carriers, and the dual carriers of *HOXB13* G84E and *CIP2A* R229Q (vs. non-carriers) with clinical characteristics are presented in Table 4. No evidence of association of *CIP2A* R229Q carrier status and clinical features of examined PrCa patients was found.

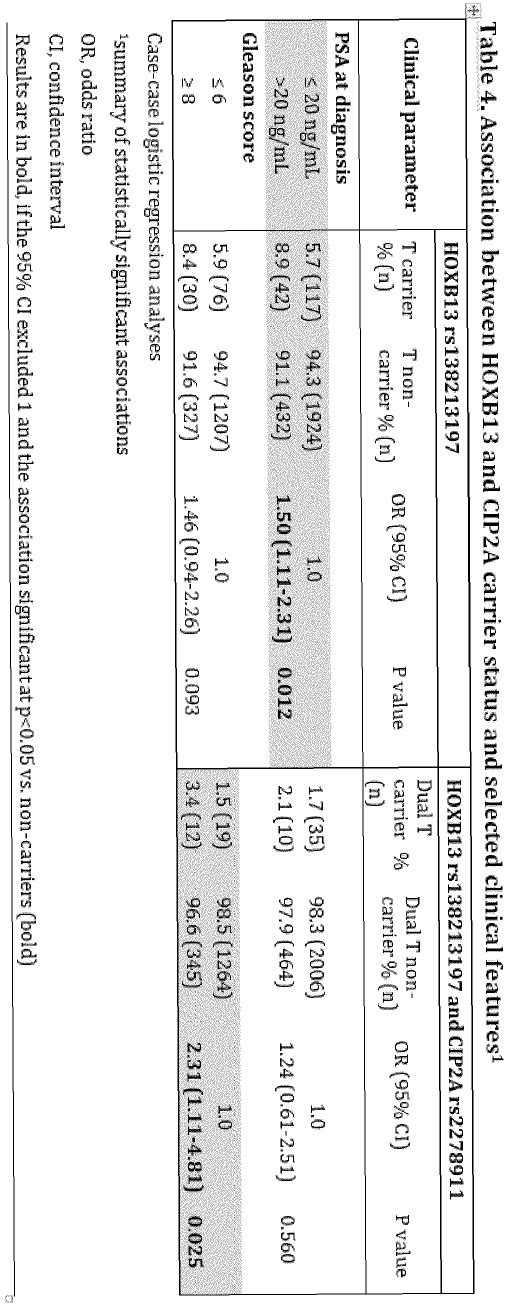

Significant association was observed between the *HOXB13* mutation status and high PSA at diagnosis (PSA>20 ng/mL; OR 1.5; 95% CI 1.1-2.3; p=0.012); total PSA data were available for 94.3% of cases. No significant association was observed between the mutation status and Gleason score presented (p=0.093).

Clinical features of dual *HOXB13* G84E and *CIP2A* R229Q carriers were examined compared with non-dual T carriers. Significant association was found between dual T carriers of *HOXB13* and *CIP2A* variants and a high Gleason score (Gleason ≥ 8, OR 2.3; 95% CI 1.1-4.8; p=0.025), which is a commonly used clinical parameter defining aggressive, poorer prognosis predicting cancer.

### Example 6. Association with specific prostate cancer subgroups

Applied case-control and direct subgroup comparison analyses of clinically differentiated PrCa and the *HOXB13* G84E, *CIP2A* R229Q carrier status revealed associations with several hallmarks of the disease (Table 5.).

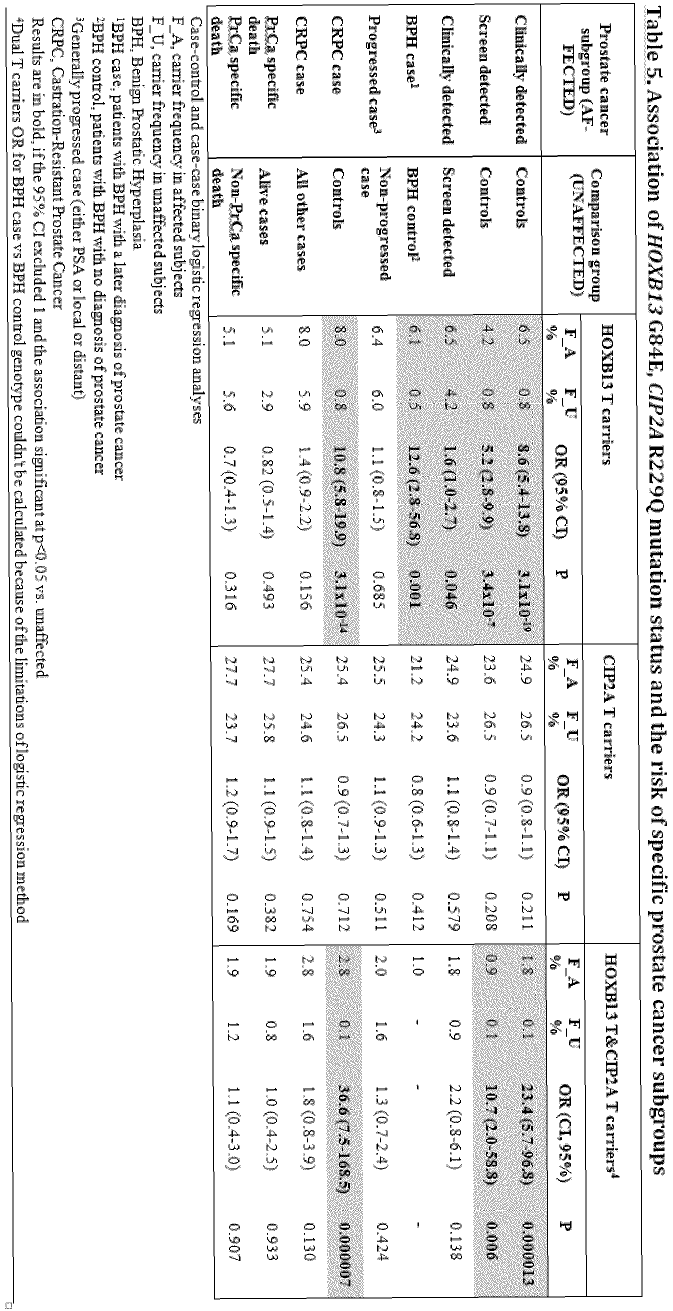

*HOXB13* mutation showed strong association with clinically detected PrCa cases (OR 8.6; 95% CI 5.4-13.8; p=3.1x10-19) vs. controls, and lower association with screen-detected PrCa vs. controls (OR 5.2; 95% CI 2.8-9.9 p=3.4x10-7). *HOXB13* T allele's role in clinically more severe disease was confirmed when clinically detected cases vs. screen detected PrCa were compared (OR 1.6; 95% CI 1.0-2.7 p=0.046). Although BPH is not considered as premalignant lesion, the present study showed that men with G84E variant with prior BPH diagnosis have 12.6-fold risk to develop PrCa compared to BPH controls (BPH, but no later development of PrCa; 95% CI 2.8-56.8; p=0.001). *HOXB13* T carrier status conferred for increased risk of PrCa in men with prior BPH diagnosis compared to men with BPH but with no later development of PrCa (OR 12.6; 95% CI 2.8-56.8 p=0.001). Importantly, statistically significant evidence of HOXB13 T allele's contribution to CRPC risk compared to controls was detected (OR 10.8; 95% CI 5.8-19.9; p=3.1x10-14).

Dual T carriers of *HOXB13* and *CIP2A* had higher risk of clinically detected prostate cancers compared with controls (OR, 23.4; 95% CI 5.7-96.8, p=0.000013) and a lower chance to be recognized through PrCa screening vs. controls (OR 10.7; 95% CI 2.0- 58.8; p=0.006). The risk for CRPC vs. controls was more than three times higher (OR 36.6 (95% CI 7.5-168.5), p=0.000007) when the patient was dual T carrier of *HOXB13* and *CIP2A* compared to patients having only the *HOXB13* T allele.

Even though the association of dual T carriers and PrCa development in BPH diagnosed men could not be statistically evaluated because of the small sample size, it is noteworthy to mention that from 590 BPH samples two were dual T carriers and they both had PrCa diagnosis. Especially important was to evaluate the risk of studied variants on development of castration resistance during the treatment, i.e., the risk of the subjects to develop castration-resistant prostate cancer.

After thoroughly examined treatment modalities, we found that patients treated with prostatectomy had a higher mutation carrier rate for *HOXB13* T, *CIP2A* T, and dual T carriers (7.4%, 25.2%, 2.2%, respectively) compared to patients on active surveillance (5.5%, 21.7%, 1.6%, respectively). Carriers of the *HOXB13* and/or *CIP2A* T alleles were hence more likely to receive primary oncological treatment.

### Example 7. Survival

In order to explore the role of *HOXB13* and *CIP2A* in overall and in PrCa specific survival, *Kaplan-Meier survival analyses* were applied. Survival time (years) was compared between *HOXB13* and *CIP2A* T carriers and those without the mutations.

Exploring the longest follow up time (birth-death), dual T carriers of *HOXB13* and *CIP2A* had significantly worse survival and they died earlier of PrCa (Breslow test, HR 3.9; 95% CI 91.6-94.5; p=0.048) vs. non dual T carriers.

The combination of *HOXB13* T and *CIP2A* T alleles seemed to have definite effect on PrCa risk, time to develop the disease, aggressive disease, survival and PrCa specific death.

### Example 8. In silico pathogenicity prediction

To further explore the function of *HOXB13* G84E and *CIP2A* R229Q variants in PrCa, and to investigate the pathogenicity prediction of G84E and R229Q variants, an *in silico* tool for missense variants, named Combined Annotation Dependent Depletion (CADD) prediction software, was used. This enables the objective integration of many diverse annotations into a single measure (C-score) for each variant. This software integrates diverse genome annotations, using altogether ∼63 different tools, applying conservation matrices as well as protein based matrices. The basis of CADD is to contrast the annotations of fixed alleles in humans with those of simulated variants. A variant is predicted to be pathogenic if the scaled C-score calculated by the software is above 10, which indicates the top 10% deleterious state among possible substitutions. For comparison the Human Genome version 37p13 (hg19) was used.

*HOXB13* G84E was predicted to be deleterious with a scaled C-score of 22.7, which indicates a potentially causative mutation. *CIP2A* R229Q variant had a scaled C-score of 21.9, denoting a similarly deleterious variant as well as a mutation potentially predisposing to cancer.

## Claims

1. A method of analyzing a subject for prostate cancer-associated markers, wherein the method comprises testing a sample obtained from said subject for *HOXB13* rs138213197 and *CIP2A* rs2278911, and wherein the presence of at least one *HOXB13* rs138213197 T allele and at least one *CIP2A* rs2278911 T allele indicates that said subject has an increased risk of having or developing prostate cancer.

2. The method according to claim 1, wherein said risk indicates earlier than an average onset of clinically relevant prostate cancer.

3. The method according to claim 1 or 2, wherein said subject has an earlier diagnosis of benign prostate hyperplasia.

4. The method according to any one of claims 1-3, wherein said subject is a Caucasian male.

5. The method according to any one of claims 1-4, wherein said method is used for a purpose selected from the group consisting of determining a subject's risk of prostate cancer, determining a subject's susceptibility to prostate cancer, determining a subject's risk of having or developing prostate cancer, diagnosing prostate cancer in a subject, prognosing prostate cancer in a subject, stratifying a subject into a high-risk or low-risk group for prostate cancer, stratifying a subject for monitoring of onset of prostate cancer, stratifying a subject for active surveillance for the progression of prostate cancer, stratifying a subject for radiotherapy or prostatectomy, stratifying a subject for clinical studies, and population screening for prostate cancer.

6. The method according to any one of claims 1-5, wherein said prostate cancer is aggressive prostate cancer.

7. The method according to claim 6, wherein the aggressive prostate cancer is castration-resistant prostate cancer.

8. The method according to any one of claims 1-7, wherein said testing for *HOXB13* rs138213197 and *CIP2A* rs2278911 is carried out at gene-level, mRNA-level, or protein-level.

9. The method according to any one of claims 1-8, which further comprises detecting one or more additional prostate cancer-associated markers selected from the group consisting of total prostate specific antigen (tPSA), free prostate specific antigen (fPSA), intact prostate specific antigen (iPSA), proPSA, human kallikrein 2 (hK2), microseminoprotein-beta (MSMB), macrophage inhibitory cytokine-1 (MIC1), prostate cancer antigen 3 (PCA3), TMPRSS2-ERG gene fusion product, ERG protein, PTEN, RAF, BRAF, SPOP, EZH2, Spink1, other prostate cancer-associated genetic markers, other genetic polymorphisms in *HOXB13* or *CIP2A* than rs138213197 or rs2278911, respectively, gene regulating factors such as eQTLs, transcription factors, enhancers, and methylation marks, biologically activated or inactivated derivatives or genetic variants thereof, and any combinations thereof.

10. The method according to any one of claims 1-9, which further comprises detecting one or more clinical variables selected from the group consisting of age, family history, previous prostate biopsy, previous diagnosis with benign prostate hyperplasia, results of biparametric magnetic resonance imaging (MRI), results of digital rectal examination (DRE), and body mass index.

11. Use of a kit in the method according to any one of claims 1-10, wherein said kit comprises a first binding body for testing said sample for *HOXB13* rs138213197 and a second binding body for testing said sample for *CIP2A* rs2278911.

12. The use according to claim 11, wherein said first binding body is for specifically determining the presence or absence of *HOXB13* rs138213197 (T) and/or said second binding body is for specifically determining the presence or absence of *CIP2A* rs2278911 (T).

13. The use according to claim 11 or 12, wherein said first and second binding bodies are selected, independently from each other, from the group consisting of nucleic acid primers, nucleic acid probes, aptamers, antibodies or antibody fragments, peptides, receptors, and enzymes such as restriction enzymes.

## Patentansprüche

1. Verfahren zur Analyse eines Probanden auf mit Prostatakrebs assoziierte Marker, wobei das Verfahren ein Testen einer Probe, die von dem Probanden erhalten wurde, auf *HOXB13* rs138213197 und *CIP2A* rs2278911 umfasst, und wobei das Vorliegen von mindestens einem *HOXB13*-rs138213197-T-Allel und mindestens einem *CIP*2A-rs2278911-T-Allel angibt, dass der Proband ein erhöhtes Risiko aufweist, Prostatakrebs zu haben oder zu entwickeln.

2. Verfahren nach Anspruch 1, wobei das Risiko ein Auftreten von klinisch relevantem Prostatakrebs, das früher als durchschnittlich ist, angibt.

3. Verfahren nach Anspruch 1 oder 2, wobei der Proband eine frühere Diagnose von benigner Prostatahyperplasie aufweist.

4. Verfahren nach einem der Ansprüche 1-3, wobei der Proband ein kaukasischer Mann ist.

5. Verfahren nach einem der Ansprüche 1-4, wobei das Verfahren für einen Zweck verwendet wird, der aus der Gruppe bestehend aus Bestimmen eines Risikos für Prostatakrebs eines Probanden, Bestimmen einer Anfälligkeit für Prostatakrebs eines Probanden, Bestimmen eines Risikos für ein Aufweisen oder Entwickeln von Prostatakrebs eines Probanden, Diagnostizieren von Prostatakrebs bei einem Probanden, Prognostizieren von Prostatakrebs bei einem Probanden, Stratifizieren eines Probanden in eine Gruppe mit hohem Risiko oder mit niedrigem Risiko für Prostatakrebs, Stratifizieren eines Probanden für eine Beobachtung auf ein Auftreten von Prostatakrebs, Stratifizieren eines Probanden für eine aktive Überwachung auf die Progression von Prostatakrebs, Stratifizieren eines Probanden für eine Radiotherapie oder Prostatektomie, Stratifizieren eines Probanden für klinische Studien und Screenen einer Population auf Prostatakrebs ausgewählt ist.

6. Verfahren nach einem der Ansprüche 1-5, wobei der Prostatakrebs aggressiver Prostatakrebs ist.

7. Verfahren nach Anspruch 6, wobei der aggressive Prostatakrebs kastrationsresistenter Prostatakrebs ist.

8. Verfahren nach einem der Ansprüche 1-7, wobei das Testen auf *HOXB13* rs138213197 und *CIP2A* rs2278911 auf Gen-Ebene, mRNA-Ebene oder Protein-Ebene durchgeführt wird.

9. Verfahren nach einem der Ansprüche 1-8, das weiterhin ein Nachweisen von einem oder mehreren zusätzlichen, mit Prostatakrebs assoziierten Markern umfasst, die aus der Gruppe bestehend aus totalem prostataspezifischem Antigen (tPSA), freiem prostataspezifischem Antigen (fPSA), intaktem prostataspezifischem Antigen (iPSA), proPSA, humanem Kallikrein 2 (hK2), Mikroseminoprotein-beta (MSMB), Makrophagen-inhibitorischem Zytokin-1 (MIC1), Prostatakrebs-Antigen 3 (PCA3), TMPRSS2-ERG-Genfusionsprodukt, ERG-Protein, PTEN, RAF, BRAF, SPOP, EZH2, Spink1, anderen mit Prostatakrebs assoziierten genetischen Markern, anderen genetischen Polymorphismen in *HOXB13* oder *CIP2A* als rs138213197 bzw. rs2278911, Genregulierungsfaktoren, wie eQTL, Transkriptionsfaktoren, Enhancer und Methylierungsmarkierungen, biologisch aktivierten oder inaktivierten Derivaten oder genetischen Varianten davon und beliebigen Kombinationen davon ausgewählt sind.

10. Verfahren nach einem der Ansprüche 1-9, das weiterhin ein Nachweisen von einer oder mehreren klinischen Variablen umfasst, die aus der Gruppe bestehend aus Alter, Familienanamnese, einer früheren Prostatabiopsie, einer früheren Diagnose mit benigner Prostatahyperplasie, Ergebnissen einer biparametrischen Magnetresonanztomographie (MRT), Ergebnissen einer digitalen rektalen Untersuchung (DRU) und Body-Mass-Index ausgewählt sind.

11. Verwendung eines Kits in dem Verfahren nach einem der Ansprüche 1-10, wobei das Kit einen ersten Bindungskörper zum Testen der Probe auf *HOXB13* rs138213197 und einen zweiten Bindungskörper zum Testen der Probe auf *CIP2A* rs2278911 umfasst.

12. Verwendung nach Anspruch 11, wobei der erste Bindungskörper zum spezifischen Bestimmen des Vorliegens oder des Fehlens von *HOXB13* rs138213197 (T) ist und/oder der zweite Bindungskörper zum spezifischen Bestimmen des Vorliegens oder des Fehlens von *CIP2A* rs2278911 (T) ist.

13. Verwendung nach Anspruch 11 oder 12, wobei der erste und der zweite Bindungskörper unabhängig voneinander aus der Gruppe bestehend aus Nukleinsäureprimern, Nukleinsäuresonden, Aptameren, Antikörpern oder Antikörperfragmenten, Peptiden, Rezeptoren und Enzymen, wie Restriktionsenzymen, ausgewählt sind.

## Revendications

1. Procédé d'analyse d'un sujet pour des marqueurs associés à un cancer de la prostate, lequel procédé comprend le test d'un échantillon obtenu auprès dudit sujet pour *HOXB13* rs138213197 et *CIP2A* rs2278911, dans lequel la présence d'au moins un allèle T de *HOXB13* rs138213197 et d'au moins un allèle T de *CIP2A* rs2278911 indique que ledit sujet présente un risque accru d'avoir ou de développer un cancer de la prostate.

2. Procédé selon la revendication 1, dans lequel ledit risque indique plus tôt que l'apparition moyenne d'un cancer de la prostate cliniquement pertinent.

3. Procédé selon la revendication 1 ou 2, dans lequel ledit sujet a un diagnostic précoce d'hyperplasie prostatique bénigne.

4. Procédé selon l'une quelconque des revendications 1 à 3, dans lequel ledit sujet est un mâle caucasien.

5. Procédé selon l'une quelconque des revendications 1 à 4, lequel procédé est utilisé dans un but choisi dans le groupe constitué par la détermination du risque de cancer de la prostate chez un sujet, la détermination de la susceptibilité d'un cancer de la prostate chez un sujet, la détermination du risque d'avoir ou de développer un cancer de la prostate chez un sujet, le diagnostic d'un cancer de la prostate chez un sujet, le pronostic d'un cancer de la prostate chez un sujet, la stratification d'un sujet dans un groupe à haut risque ou à faible risque de cancer de la prostate, la stratification d'un sujet pour surveiller l'apparition d'un cancer de la prostate, la stratification d'un sujet pour une surveillance active de la progression d'un cancer de la prostate, la stratification d'un sujet pour une radiothérapie ou une prostatectomie, la stratification d'un sujet pour des études cliniques, et le criblage d'une population pour un cancer de la prostate.

6. Procédé selon l'une quelconque des revendications 1 à 5, dans lequel le cancer de la prostate est un cancer de la prostate agressif.

7. Procédé selon la revendication 6, dans lequel le cancer de la prostate agressif est un cancer de la prostate résistant à une castration.

8. Procédé selon l'une quelconque des revendications 1 à 7, dans lequel ledit test pour *HOXB13* rs138213197 et *CIP2A* rs2278911 est mis en œuvre au niveau génique, au niveau de l'ARNm, ou au niveau protéique.

9. Procédé selon l'une quelconque des revendications 1 à 8, qui comprend en outre la détection d'un ou plusieurs marqueurs associés à un cancer de la prostate additionnels choisis dans le groupe constitué par l'antigène spécifique de la prostate total (tPSA), l'antigène spécifique de la prostate libre (fPSA), l'antigène spécifique de la prostate intact (iPSA), le proPSA, la kallikréine 2 humaine (hK2), la microséminoprotéine bêta (MSMB), la cytokine 1 inhibant les macrophages (MIC1), l'antigène 3 de cancer de la prostate (PCA3), le produit de fusion génique TMPRSS2-ERG, la protéine ERG, PTEN, RAF, BRAF, SPOP, EZH2, Spink1, d'autres marqueurs génétiques associés à un cancer de la prostate, d'autres polymorphismes génétiques dans *HOXB13* ou *CIP2A* autres que rs138213197 ou rs2278911, respectivement, des facteurs de régulation génique tels que les eQTL, des facteurs de transcription, des amplificateurs, et des marqueurs de méthylation, des dérivés ou variants génétiques biologiquement activés ou inactivés de ceux-ci, et l'une quelconque de leurs combinaisons.

10. Procédé selon l'une quelconque des revendications 1 à 9, qui comprend en outre la détection d'une ou plusieurs variables cliniques choisies dans le groupe constitué par l'âge, l'historique familial, une biopsie antérieure de la prostate, un diagnostic antérieur d'une hyperplasie prostatique bénigne, les résultats d'une imagerie par résonance magnétique (IRM) biparamétrique, les résultats d'un toucher rectal (DRE), et l'indice de masse corporelle.

11. Utilisation d'une trousse dans le procédé de l'une quelconque des revendications 1 à 10, dans laquelle ladite trousse comprend un premier corps de liaison pour tester ledit échantillon pour *HOXB13* rs138213197 et un deuxième corps de liaison pour tester ledit échantillon pour *CIP2A* rs2278911.

12. Utilisation selon la revendication 11, dans laquelle ledit premier corps de liaison est pour déterminer spécifiquement la présence ou l'absence de *HOXB13* rs138213197 (T) et/ou ledit deuxième corps de liaison est pour déterminer spécifiquement la présence ou l'absence de *CIP2A* rs2278911 (T) .

13. Utilisation selon la revendication 11 ou 12, dans laquelle lesdits premier et deuxième corps de liaison sont choisis, indépendamment l'un de l'autre, dans le groupe constitué par les amorces d'acide nucléique, les sondes d'acide nucléique, les aptamères, les anticorps ou fragments d'anticorps, les peptides, les récepteurs, et les enzymes telles que les enzymes de restriction.
